Europäisches Patentamt

European Patent Office

Office européen des brevets

(19)

(11) Publication number: **0 636 383 A1**

## EUROPEAN PATENT APPLICATION

(12)

(21) Application number: **94109963.2**

(22) Date of filing: **28.06.94**

(51) Int. Cl.6: **A61M 25/00**, A61M 25/01, A61B 17/00

(30) Priority: **30.06.93 JP 161700/93**

(43) Date of publication of application:
**01.02.95 Bulletin 95/05**

(84) Designated Contracting States:
**CH DE FR LI NL**

(71) Applicant: **Ishimura, Takao**
**302, 16-17, Sumiyoshi Honcho 3-chome,**
**Nada-ku**
**Kobe-shi,**
**Hyogo-ken (JP)**
Applicant: **Fuji Systems Kabushiki Kaisha**
**23-14, Hongo, 3-chome,**
**Bunkyo-ku**
**Tokyo (JP)**

(72) Inventor: **Ishimura, Takao**
**302, 16-17, Sumiyoshi Honcho 3-chome,**
**Wada-ku**
**Kobe-shi,**
**Hyogo-ken (JP)**
Inventor: **Mitsuhashi, Hiroyuki**
**2754-20, Kawawa-cho,**
**Midori-ku**
**Yokohama-shi,**
**Kanagawa-ken (JP)**

(74) Representative: **Weiss, Peter, Dr. rer. nat.**
**Patentanwalt,**
**Zeppelinstrasse 4**
**D-78234 Engen (DE)**

(54) **Method of angiography.**

(57) The disclosed is a method of angiography which reduces the burden beared by the patient due to the surgery. By replacing catheters and by means of brachial paracentesis, angiography is performed on each of coronary artery, vein bypass, gastroepiploic artery and internal thoracic artery, through the steps of: providing puncture of a paracentetic tube from a brachium, and, through the tube above, inserting a catheter selected from the following group: (a) a catheter of the type in use for the left and right coronary artery and the venous bypass; (b) a catheter of the type in use for the inernal thoracic artery; and (c) a catheter of the type in use for the gastroepiploic artery.

*FIG.1*

*FIG.2*

EP 0 636 383 A1

# FIG.3

2

BACKGROUND OF THE INVENTION

1. Field of the Invention

The present invention relates to a method of angiography of coronary arteries, vein bypasses, gastroepiploic arteries and internal thoracic arteries.

2. Description of the Prior Art

In accordance with the increasing cases of percutaneous angiography or coronary arterial bypass surgeries, cases of repeating coronary arterial and bypass angiography have increased. Also, in the western countries, for the purpose of cost saving of medical expenses, there have been increasing cases for performing coronary artery angiography in outpatient sites.

Conventionally, coronary artery angiography has employed two methods including "Judkins" method comprising the step of puncture of a paracentetic tube into the femoral artery, from which a catheter is inserted: and "Sones" method comprising the step of incising the skin of the brachial region to expose the artery, to which a hole is formed to be inserted by a catheter.

Advantages and disadvantages of the prior art are as follows. In Jadkins method, performed only by puncture of a paracentetic tube into a blood vessel, compared to Sones method performed by incision of the vessel, damage of skins and vessels is minimized. Also, insertion of a catheter from the paracentetic tube into the blood vessel minimizes damage of blood vessel caused by entrance and exit of the catheter.

Since the process is performed without incision of cortex and blood vessel, no wound or adhesion of wound region remains. Therefore, even in angiography which requires frequent repetition of angiographic processes, it is advantageously possible to repeat such processes from the same region.

In Judkins' method, (1) the catheter used in this method is in advance formed to easily enter the coronary artery, and to be operated by a beginner through a relatively short term training.

(2) On the other hand, one of disadvantages of Judkins' method is that the patient would feel ashamed because the catheter is inserted from the femoral region.

However, because of the paracentetic tube being punctured, after removal of the tube, often bleeding occurs, and it is required a long time pressurising and laying quiet for hemostasis. Therefore, the patient suffers from burden.

(3) For angiography of the left and right coronary arteries and venous bypass, a separate catheter is employed, respectively, and it is necessary to replace the catheter, thereby demanding necessary time and costs as well for performing angiography.

(4) In most western countries, for the purpose of cost saving of medical expenses, although there are increasing cases for performing coronary artery angiography in outpatient sites or mobile angiography in automobiles, it is difficult to perform the Judkins' method as reasons mentioned in (2) and (3).

(5) This method cannot be employed for cases of arterial obliteration, such as stenosis in the gastroepiploic artery or femoral artery.

In Sones' method, the brachial region is incised to expose the artery to which is formed an opening, through which a catheter is inserted. Thus, by using a brachium, the patient does not feel ashamed.

After an angiograph is formed, the opening is seamed. As the incised region is in brachium, the quiet time required after angiography may be one hour or so, thereby the the patient receiving less load.

Also, only one catheter is used for forming angiograph of both the right coronary artery and venous bypass.

On the other hand, one of disadvantages of the Sones' method is that, since the cortex is incised to form an opening, through which a catheter is inserted, the patient suffers from the burden and the time necessary for his operation. Also, the incised cortex causes the vessel to expose and leave wound and adhesion. As a result, the incision cannot be done at the same region, and repeated angiographic operation must be done in shifted positions, thereby the region to be incised is limited in repeated cases. In addition, the techniques of cutaneous incision or insertion of the catheter into the coronary artery or bypass are so difficult to require a long time period such as normally one or two years for a person to acquire the technique. Also in most western countries, for the purpose of cost saving of medical expenses, although there are increasing cases for performing coronary artery angiography in outpatient sites, it is difficult to perform the Sones' method as reasons mentioned above.

## SUMMARY OF THE INVENTION

The present invention is to provide a method of angiography of any of a coronary artery, a venous bypass, a gastroepiploic artery and an internal thoracic artery by means of brachial paracentesis comprising the steps of: puncture from the paracentetic tube an improved thin catheter of the following a, b or c as follows:

(a) a catheter of the type "a" in use for the left and right coronary artery and the venous bypass;

(b) a catheter of the type "b" in use for the internal thoracic artery; and

(c) a catheter of the type "c" in use for the gastroepiploic artery.

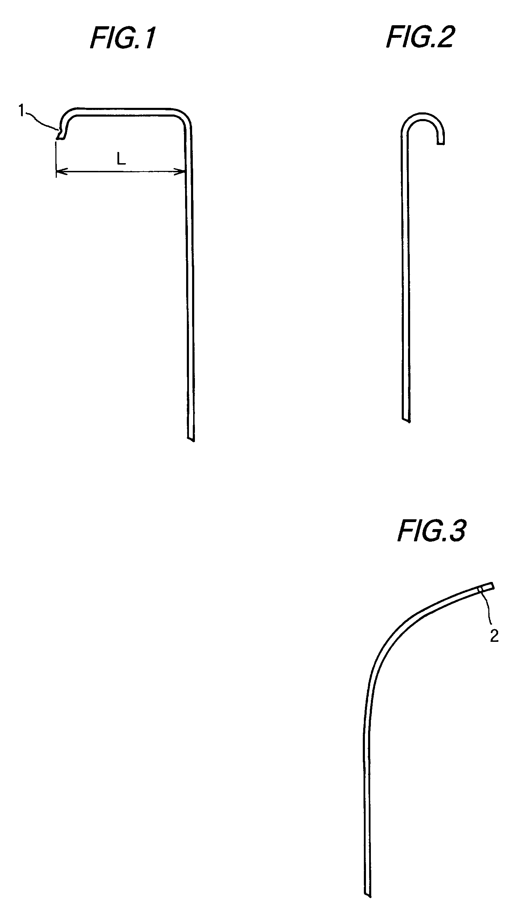

The catheters above are, as shown in FIG. 1, those manufactured, for example, by the applicants, as Interbeck type heart-use catheters.

## BRIEF DESCRIPTION OF THE DRAWINGS

FIG.1 is a front view of a catheter of type "a";

FIG.2 is a front view of a catheter of type "b"; and

FIG.3 is a front view of a catheter of type "c".

## DETAILED DESCRIPTION OF THE INVENTION

Type "a" of the catheter, to facilitate a rotational operation, is thinned as 2Fr and 5.2Fr, 800 mm in length, and 35 mm and 40 mm in the end length L. The tip end thereof is a soft tip, and formed with an opening. In addition, one or two of side holes 1 of 0.1 to 1.5 mm in diameter are formed at the position of 2 to 10 mm from the tip (not shown) to provide increased safety, and also provided with a circular section to facilitate flow of the angiographic agent.

Type "b" of the catheter is designed to be inserted from the brachium, which is properly used for performing an internal thoracic arterial angiography, formed with its length of 800 mm, and can be easily and most electively inserted within the internal thoracic artery.

Type "c" of the catheter is designed to be also inserted from the brachium, which is properly used for performing a gastroepiploic arterial angiography, and 800 mm in length. Its one end is open, on the side of which a through hole 2 of 0.1 to 1.5 mm in diameter is formed at the position of 2 to 20 mm apart from the tip end. It is shaped similar to the Sones' catheter, but is formed to have the length and shape of its tip end conformed to easily engage with the exit of the femoral artery. In addition, by using a guide wire, this catheter can be properly and most electively inserted within the gastroepiploic artery.

The comparison between the angiography according to the invention and those of Judkins' and Sones' methods is shown in the Table 1.

Table 1: Comparison between Methods of Angiography

| Sones' Method | Judkins' Method | Invention's Method |
|---|---|---|
| Approached from brachial artery. | Approached from right/left femoral artery. | Approached from right/left brachial artery. |
| Percutaneous incisision. Using Sones' catheter. | Paracentesis. Judkins' catheter. | Paracentesis. Inventors' catheters for left/right coronary artery and venous bypass. |
| Without replacing of catheter, angiograph of right & left coronary artery and left ventricle. | Separate catheter is necessary for left and right coronary artery and left ventricle, each. | Without replacing the catheter, left/right coronary arteries are enabled. Separate catheter is necessary for ventricle angiography. |
| Training needed for catheter operation. | Operation of catheter is relatively easy. | Operation of catheter is relatively easy. |
| Surgical techniques are needed. | Surgical techniques are unnecessary. | Surgical techniques are unnecessary. |

| | | |
|---|---|---|
| Insertion opening for catheter is closed by sutration, and repeated insertion through the same region is impossible. | Debleeding by pressurizing. | Debleeding by pressurizing. No incision allows repeated insersion of catheter throu-gh the same region. |
| Almost no hematoma or bleeding, but may cause arterial obliteration due to thrombus. | Possibility of hematoma or after-bleeding. | Minimized possibility of hematoma or after-bleeding. |
| After operation, requiring extension of bracium, and brachial relaxation for about an hour. | Needed rest quiet in bed is 12 to 24 hours after operation. | Needed rest quiet is only an hour in bed and 3 hours for brachial relaxation. |
| Principally, 8 Fr catheter is used. | Principally, 6 Fr catheter is used. | Principally, 5 Fr and 5.2 Fr catheter are used. |

Only by puncture of a paracentetic tube into the brachial artery, only a few wound is formed on the artery. Also, the use of a thin catheter leaves only a few wound compared with the Sones' method which requires incision of the skin to expose the artery, thereby the load beared by the patient being minimized.

Because of a brachial artery which is punctured by the paracentetic tube, even though the patient moves after removing the tube, bleeding caused by being pressurized hardly occurs. It is required only one hour or so for the patient to take quiet rest, also minimizing his burden.

For the reason that the distance from the brachium to the heart is greater than that between the femoral region and the heart, a short catheter can be used, providing preferred operability and requiring largely shortened time for angiographic inspection. In practice, only a halved time is needed compared with that needed in insertion through the femoral region, providing the patient with a minimized burden.

For reasons mentioned above, the prevent invention is suitable for the cases which require repeated angiographic operations.

Examples of Cases

Example 1: A patient Y.K., 70 years old.

In 1966: hospitalized owing to crisis of myocardial infarction. Since 1987, an intermittent claudication as an arterial obliteration occurred.

In December 1987: angiography performed for the first time and hospitalized for 10 days. As a result, stenoisis was found in the coronary artery, and the abdominal aorta was completely obliterated in its lower region. The time needed for inspection was 65 minutes, that for X-ray examination was 20.4 min., and the rest quiet time after operation was one hour.

In November 1991: The coronary arterial angiography of the second time was performed for review of the proceeding and paracentesis. Hospitalized for 3 days. The inspection having completely the same contents as those in the first inspection was performed as follows: pressing the right branchium, inserting a paracentetic tube, using a catheter of the inventors' type prepared in use for right and left coronary arteries and a conventional 5-inch pigtail catheter. The time required for examination was 45 minutes, which were remarkably compared with those at the first operation. Together with one hour for rest, the burden beared by the patient was clearly reduced. The case was that could not be made from the femoral artery by Judkins' method because of the obliterated abdominal aorta.

Example-2 : A patient T.F., 62 years old.

Since 1985: crisis of stenocardia.

In January 1985 and January 1988, respectively: the first and second times of coronary angiography performed by Sones' method through incision of the right brachium to the artery.

In March 1993: the third coronary angiography was performed through incision of the right brachium and using an inventors' catheters for use in right and left coronary arteries, where the necessary time for examination was 42 minutes, roentgenoscopy was 7.2 min., thus each time was remarkably reduced compared with that of the first and second operations.

Thereafter, the lesion was worsened, and operations of the coronary arterial bypass surgery were performed on three regions, including those on the venous bypass, the left internal thoracic arterial bypass, and the gastroepiploic artery.

In June 1993: another coronary angiography was performed for confirming the existence of the openness of the bypass after the previous operation, with the patient as being hospitalized after operation. The angiography was performed: (a) in the left and right coronary arteries and vein bypass, using an inventors' type catheter of left and right coronary arterial use; (b) in the internal thoracic artery and venous bypass, using a catheter of internal thoracic arterial use; and (c) in the gastroepiploic arterial bypass, using a catheter of gastroepiploic arterial use. Although the number of catheters for performing angiography was increased, yet the reduced time of including 55 min. for operation, 14.4 min. for roentgenoscopy, together with one hour for the patient to take rest after operation.

Paracentesis, the technique which has been already established, is not so difficult as the incision technique in Sones' method, and operation of the catheter is also easily acquired even by a beginner in relatively short time since it is formed in a shape easily inserted into any artery. Further, the patient would not feel ashamed using a brachial region.

Angiography of right and left coronary artery and vein bypass is performed using only one catheter. Also, angiography of the internal thoracic artery as well as that of the gastroepiploic artery, which were in the past normally performed only through the femoral region, are all performed through the brachial region by changing the catheter. In these cases, either one of the catheter, suitable for the respective artery, may be selected from the above-mentioned catheters "a", "b" and "c".

For reasons mentioned above, artery angiography in outpatient sites or mobile angiography using automobiles are also possible to be performed.

Only a small number of catheters may be used to enable angiography performed at the outpatient sites, thereby saving the costs of medical expenses.

Any case of arterial obliteration such as a clogged abdominal artery or femoral artery may be also examined.

In summary, the present invention provides the effects including: reduction of the burden on the patient who is subject to the surgery: and being used for cases requiring repeated process of angiography.

The techniques necessary for including catheters are acquired in comparatively reduced time of period.

Angiography is performed with only one catheter for the right coronary artery as well as venous bypass; and, by replacement of catheters, for the internal thoracic and gastroepiploic arteries, and all of the above can be performed through the brachial approach, and also performed in outpatient sites as well as mobile surgery using automobiles. Also, all the treatments above are of reduced cost of medical expenses.

**Claims**

1. A method of angiography of any of a coronary artery, a venous bypass, a gastroepiploic artery and an internal thoracic artery by means of brachial paracentesis, comprising the steps of:

   providing puncture of a paracentetic tube for the brachial artery; and

   inserting through said paracentetic tube an improved thin catheter selected from the following catheters:

   (a) a catheter of the type in use for the left and right coronary artery and the venous bypass;

   (b) a catheter of the type in use for the internal thoracic artery; and

   (c) a catheter of the type in use for the gastroepiploic artery.

2. A method of angiography according to claim 1, wherein said catheter of the type in use for the left and right coronary artery and the venous bypass includes an elongated tubing and a tip end substantially perpendicularly extending from one end of said tubing.

3. A method of angiography according to claim 1, wherein said catheter of the type in use for the internal thoracic artery includes an elongated tubing and a tip end arcuately extending from one end of said tubing.

4. A method of angiography according to claim 1, wherein said catheter of the type in use for the gastroepiploic artery includes an elongated tubing and a tip end extending and slightly curved from one end of said tubing.

*FIG.1*

*FIG.2*

*FIG.3*

European Patent
Office
**PARTIAL EUROPEAN SEARCH REPORT**
which under Rule 45 of the European Patent Convention
shall be considered, for the purposes of subsequent
proceedings, as the European search report

Application number

## DOCUMENTS CONSIDERED TO BE RELEVANT

EP 94109963.2

| Category | Citation of document with indication, where appropriate, of relevant passages | Relevant to claim | CLASSIFICATION OF THE APPLICATION (Int Cl6) |
|---|---|---|---|
| X | <u>US - A - 4 983 169</u><br>(Y. FURUKAWA)<br>    * Fig. 1; column 1,<br>      lines 6-20; column 5,<br>      lines 56-62 * | 3 | A 61 M 25/00<br>A 61 M 25/01<br>A 61 B 17/00 |
| X | <u>US - A - 4 581 390</u><br>(V.J. FLYNN)<br>    * Fig. 1; column 8,<br>      lines 8-24; column 11,<br>      lines 23-37 * | 3 | |
| X | <u>US - A - 5 088 991</u><br>(T.D. WELDON)<br>    * Fig. 1,2; column 1,<br>      lines 9-20; column 3,<br>      lines 50-53 * | 4 | |
| A | CORDIS "KATALOG DER ANGIO-<br>GRAPHISCHEN PRODUKTE",<br>October 1983,<br>imprint: Cordis 151-0026G<br>Rev. 4<br>pages 1-8 | 2 | **TECHNICAL FIELDS SEARCHED (Int Cl6)**<br><br>A 61 M<br>A 61 B |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 2-4

Claims searched incompletely:

Claims not searched: 1

Reason for the limitation of the search: according to Article 52(4) EPC (ad "Methods for treatment of the human... body by surgery...and diagnostic methods practised on the human ...body...")

| Place of search | Date of completion of the search | Examiner |
|---|---|---|
| VIENNA | 13-10-1994 | LUDWIG |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO Form 1505.1 03.82

| DOCUMENTS CONSIDERED TO BE RELEVANT | | | CLASSIFICATION OF THE APPLICATION (Int. Cl.6) |
|---|---|---|---|
| Category | Citation of document with indication, where appropriate, of relevant passages | Relevant to claim | |
| X | -- | 3,4 | |
| A | US - A - 4 898 591 (Y.T. JANG et al.) * Fig. 4; column 1, lines 6-13; column 6, lines 15-31 * -- | 2-4 | |
| A | EP - A - 0 148 131 (U. PASQUI) * Fig. 2; page 9, lines 8-16 * -- | 2 | |
| A | US - A - 4 694 838 (B. WIJAYARTHNA) * Fig. 1,3,5; column 1, lines 11-15; column 5, lines 60-64 * ---- | 2 | TECHNICAL FIELDS SEARCHED (Int. Cl.6) |